(19) **Europäisches Patentamt / European Patent Office / Office européen des brevets**

(11) **EP 4 675 634 A1**

(12) **EUROPEAN PATENT APPLICATION**

(43) Date of publication:
07.01.2026 Bulletin 2026/02

(21) Application number: 24185914.9

(22) Date of filing: 02.07.2024

(51) International Patent Classification (IPC):
*G16H 30/40* (2018.01)    *G16H 50/20* (2018.01)

(52) Cooperative Patent Classification (CPC):
**G16H 50/20; G16H 30/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **KROENKE-HILLE, Sven**
  **Eindhoven (NL)**
• **YOUNG, Stewart Matthew**
  **Eindhoven (NL)**

• **VON BERG, Jens**
  **5656AG Eindhoven (NL)**
• **BYSTROV, Daniel**
  **Eindhoven (NL)**
• **GOOSSEN, André**
  **Eindhoven (NL)**
• **DESHPANDE, Hrishikesh Narayanrao**
  **Eindhoven (NL)**
• **MENSER, Bernd**
  **Eindhoven (NL)**
• **BERGTHOLDT, Martin**
  **Eindhoven (NL)**

(74) Representative: **Philips Intellectual Property & Standards**
**High Tech Campus 52**
**5656 AG Eindhoven (NL)**

(54) **AUTOMATIC VISUAL GUIDANCE FOR READING MEDICAL IMAGES**

(57) A system (FRS) and related method for facilitating radiological reading of medical imagery. The system comprises an input interface (IN) for receiving input data including a medical image to be read by a user. An analyzer (AZ) identifies, based on the medical image (m), one or more critical portions (R1-3). An alert module (AM) issues an alert signal, based on the so identified one or more critical portions (R1-3), and on user generatable gaze information (g) in respect of the said medical image displayable on a display device (DD).

Fig. 2

EP 4 675 634 A1

**Description**

FIELD OF THE INVENTION

The invention relates to a system for facilitating radiological reading of medical imagery, to a medical imaging arrangement including such a system, to a training system for training a machine learning model, to a training data provider system, to related methods, to a computer program element and to a computer readable medium.

BACKGROUND OF THE INVENTION

Imaging is among the most useful tools in the medics' arsenal. It enables them to look "inside" a patient in a non-invasive manner, thereby providing valuable clues to inform therapies, diagnosis, planning or whatever the medical task at hand.

Radiologists for example are trained to read, that is to interpret and analyze, displayed imagery, in order to screen for disease or medical conditions the patient may suffer from. For example, in mammography, radiologists read in a reading session X-ray imagery of the patient's breasts, in order to find clues, such as nodules that may betoken breast cancer. Similarly, lung cancer screening, X-ray imagery ("chest X-ray") of the lungs is read for lesions or definition of suspicious masses, etc.

There appear to be three factors that combine unfavorably into overwhelming workloads for radiologists. One such factor is that medical imaging became more sophisticated producing high dimensional complex imagery with thin slices for example. It has been estimated that an average CT scan for example includes in excess of six hundred slices as reported by in study by R Alexander et al, "Mandating Limits on Workload, Duty and Speed in Radiology", published in in Radiology, vol 304, pp 274-282. Second, there is a shortage of radiologists and third there is an ever-aging population. These factors together yield unprecedented workloads, which according to the *Alexander et al* report (supra), has grown by more than 500% in some fields. Such workloads may result in user fatigue and to wrong reading conclusions, the consequences of which range from the annoying and expensive - such as therapies being meted out that were not warranted in the first place - to the catastrophic, where patients succumb to disease before their time because they did not receive the care they needed on account of reading errors.

There may thus be a need, in the imaging field, to improve image readings, in particular but not necessarily only, in the medical field.

SUMMARY OF THE INVENTION

An object of the present invention is achieved by the subject matter of the independent claims where further embodiments are incorporated in the dependent claims. It should be noted that the following described aspect of the invention equally applies to the medical imaging arrangement including such a system, to the training system for training the machine learning model, to the training data provider system, to related methods, to the computer program element and to the computer readable medium.

According to a first aspect of the invention there is provided a system for facilitating radiological reading of medical imagery, comprising:

an input interface for receiving input data including a medical image to be read by a user;
an analyzer capable of identifying, based on the medical image, one or more critical portions; and
an alert module capable of issuing an alert signal, based on the so identified one or more critical portions, and on user generatable gaze information in respect of the said medical image displayable on a display device.

In embodiments, a criticality of said one or more portions is based on reading of prior imagery by prior one or more users.

The criterion / determination of whether a pixel or voxel or other image element belongs to one of the one or more a critical portion may be based on the prior imagery, in particular the manner such prior imagery was read by the prior one or more user, who may be experts, such as in radiology. The said manner for reading may be established based on capturing gaze information of the expert(s) during their reading of the prior imagery. This, a similar setup may be used as for the current user. Preferably, the prior imagery is of the same kind or type as the input medical image, such as both may be chest imagery, abdomen imagery, head imagery, etc, Thus, the imagery should preferably pertain to the same type of anatomy, but are otherwise in general different, eg, are from different patients, or if from the same patients, were obtained in different imaging sessions.

In embodiments, the issuing of the alert signal is based on the alert module comparing the said one or more critical portions against the said gaze information.

In embodiments, the said alert module, in so comparing, is capable to consider one or more factors including any one or more of: i) gaze dwelling time over the one or more critical portions, ii) number of the one or more critical portions over the gaze information.

Thus, based on the current user's gaze information and the critical image portions, the alert module may apply a quality check or other policy in respect of the current user's reading session. If this policy is violated, the alert signal, in whichever form, may be issued. This policy may include how long current user may have looked at one or more of the critical portions, and/or how many of the critical portions the user has looked at, etc. Any policy may be used herein that is capable of measuring/quantifying how current user is paying attention to some, preferably all, of the critical portions. As the critical portions represent an estimate for the way or manner the experts would interpret such an image, in particular which part of the imagery such expert would have examined, the said comparing can be used for such quantification. The policy may be based on baseline data as may be aggregated from one or more of the prior users.

In embodiments, the analyzer is based on a trained machine learning model.

In embodiments, the at least one or more of the said identified one or more critical portions is displayable on the, or a, display device.

In embodiments, the gaze information is acquirable by a tracker system.

In embodiments, the tracker system includes a headset wearable by user during reading.

In another aspect there is provided an arrangement comprising a system of any one of preceding aspect or embodiment, and further comprises any one of more of: i) display device, ii) the tracker system, iii) an imaging apparatus (IA) providing the medical image, iv) a database from which the medical image is retrievable.

In another aspect there is provided a machine learning training system or training, based on training data, the machine learning model.

In another aspect there is provided a training data provider system capable of providing the training data for training of the machine learning model by training system.

In embodiments, the training data provider system is capable of capturing gaze information from prior one or more users, whilst said prior one or more users view prior imagery, the said gaze information forming part of the training data.

The providing of training data may include data cleansing, such as saccade trace removal or other.

In another aspect there is provided a method for facilitating radiological reading of medical imagery, comprising:

receiving input data including a medical image to be read by a user;
identifying, based on the medical image (m), one or more critical portions; and
issuing an alert signal, based on the so identified one or more critical portions, and on user generatable gaze information in respect of the said medical image displayable on a display device.

In another aspect there is provided a method of training, based on training data, the machine learning model.

In another aspect there is provided a method of providing training data for training the model by the method of training.

In another aspect there is provided a computer program element, which, when being executed by at least one processing system, is adapted to cause the processing system to perform the method for facilitating radiological reading of medical imagery, or the method of training, or method of providing training data.

In another aspect there is provided at least one computer readable medium having stored thereon the program element, or having stored thereon the trained machine learning model or parameters thereof.

The proposed setup allows addressing the effect of an ever-increasing number of medical images to be read, combined with many hospitals facing a severe shortage of experienced staff radiologists. This may cause stress due to high workload and quality issues. Training up new radiologists ordinarily takes time so that no relief of the situation is to be expected soon. The proposed setup facilitates more efficient training of new radiologists, for example.

The proposed system may take a medical image as an input and may use a machine learning algorithm to predict which regions an experienced radiologist would pay attention to. Such regions may be display in some visualization to the (novice) radiologist reading the medical image at hand. The proposed system can be used in a quality-supervision system, for reading radiologists: the attention map is predicted for the image to be read, and whilst the radiologist (novice or expert) is reading the image, he / she is monitored by an eye tracker for example. The resulting gaze points / trajectory may be post-processed, such as by removal of saccade points. The so cleaned gaze points may then be compared to the predicted attention map: If there are no or too few gaze points in a region of high predicted attention values, an alert (such as a visual highlighting of the relevant region) is emanated such as by displaying or any other to the reading radiologists so that he / she can check whether some findings may have been missed. Such alert may include visual highlighting of the relevant region.

The proposed system and method can be applied to medical images of any modality. It may be of benefit, in particular for medical images of large size / large field-of-view. The system can be integrated into a PACS viewer or used in training software for radiologists.

In the following when reference is made to such image elements, we shall refer to pixels primarily, however, this is not to the exclusion of voxels or other more coarse-grained elements (chunking) as may be called for in certain circumstances. Thus, a reference herein to "pixels" is synecdochally and thus not limiting but is merely a convenient shorthand. Having said that, the principles described herein may well be used for 2D pixel data (such as in radiography). Thus, such principles are not confined to high-dim volumetric /tomographic data, although the benefits of the proposed system may be most keenly felt there on account of the complex data structure of such tomographic imagery.

In general, the herein described processing of data - be it in deployment or training - is not confined to representation in spatial domain. The data may be transformed into other domains, such as f-domain, e.g. all manners of harmonic analysis (Fourier, Laplace, Wavelet, etc). Processing may occur at least in parts in such other transformed into space, with results obtainable their inverse-transformed back to spatial domain, as needed. Space invariant transformations that transforms into the same domain, such as Hilbert transform or others, are also envisaged herein.

In general, the term *"machine learning"* includes a computerized arrangement (or module) that implements a machine learning ("ML") algorithm. Some such ML algorithms operate to adjust a machine learning model, thus configuring same to perform ("learn") a task. Other ML operate direct on training data, not necessarily using such an explicit model, thus in such cases the training data may form the model or may be part of such model. This adjusting or updating of the model is called "training". In general, task performance by the ML model may improve measurably with training experience. Training experience may include suitable training data, and exposure of the model to such data. Task performance may improve, the better the data represents the task to be learned. Training experience helps improve performance if the training data well represents a distribution of examples over which the final system performance is measured. See for example, T. M. Mitchell, "Machine Learning", page 2, section 1.1, page 6 1.2.1, McGraw-Hill, 1997. The performance may be measured by objective tests based on output produced by the model in response to feeding the model with test data. The performance may be defined in terms of a certain error rate to be achieved for the given test data.

*"(Current) user"* relates to a person, such as medical personnel or other, using the proposed system for image reading. The *"expert /prior user"* may have done such reviews of other, past, imagery, in the past, and their attention data is recorded and may be used herein to train an ML model for use by the current user.

BRIEF DESCRIPTION OF THE DRAWINGS

Exemplary embodiments of the invention will now be described with reference to the following drawings, which, unless stated otherwise, are not to scale, wherein:

Exemplary embodiments of the invention will now be described with reference to the following drawings wherein:

Fig. 1 shows a schematic block diagram of a computer assisted arrangement for reading imagery, in particular in the medical realm;
Fig. 2 show a block diagram of a computerized reading facilitator system as proposed herein in some embodiments;
Fig. 3 shows a block diagram of an alert module as may be used in the system of Figure 2 according to some embodiments;
Fig. 4 shows optional visualizations of data obtainable by the system according to Figure 2;
Fig. 5 shows a flow chart of a method of facilitating the reading of imagery, particularly of medical imagery;
Fig. 6 shows a block diagram of a training system for training a machine learning model;
Fig. 7 shows a block diagram of training data provider system as may be envisaged herein in some embodiments to facilitate training a machine learning model;
Fig. 8 shows a block diagram of an architecture of a machine learning model as may be used herein in some embodiments;
Fig. 9 shows a flow chart of a method of providing training data for training a machine learning model; and
Fig. 10 shows a flow chart of a method of training a machine learning model.

DETAILED DESCRIPTION OF EMBODIMENTS

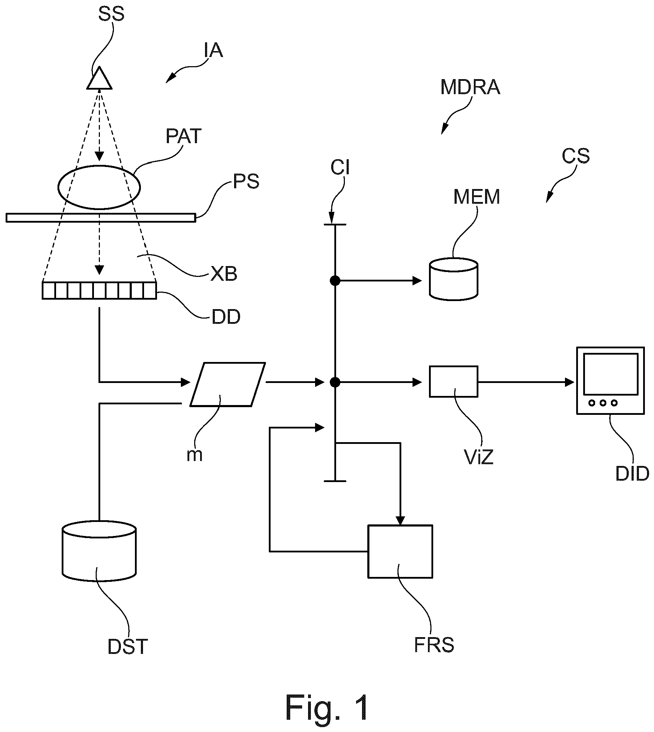

Reference is now first made to Fig. 1 which shows components of a medical data reading arrangement MDRA assisted by a computer system CS.

Broadly, the arrangement may include a data source such as a medical imaging apparatus IA that is operable to acquire imagery $m$ of a patient IA. Alternatively, or in addition, the imagery may be retrieved from an image data storage DST, such a PACS in a hospital or medical facility or other. Via wireless, wired or hybrid (wired and wireless) communication interface CI, such as a network, the imagery $m$ is passed on to the computing system CS for processing. The imagery may be stored in a memory MEM. In addition, or instead, the visualizer VIZ may be operable to effect visualization of the imagery in a graphics display displayable on a monitor DID. For example, the visualizer VIZ may map digital image information comprised in the imagery m to a grey -value or color palette, which is then used to drive video circuitry of the graphics display, thereby causing display of graphics display on screen of display device DID.

The computing system CS thus described may be a workstation or may be part of such workstation. Such workstation may be used by a user/image reviewer, such as a radiologist, in reviewing (reading) such medical imagery m in a radiological reading sessions. In order to enhance contrast and viewing experience, this may be done in a dimly lit room for example, where the radiologist is carefully reviewing the displayed imagery. The user's eyes carefully "scan" the displayed information for possibly suspicious features in order to ascertain whether there is reason for concern. For example, whether in-image structures as perceivable using reviewer's medical knowledge that may warrant further investigation, planning or even surgery, or whatever course of action may be applicable.

Broadly, as will be explored in mor detail below, the computing system CS may include, or may be capable of interfacing with, an image reading facilitator FRS that facilitates reading of imagery, or of other spatial data. Thus, image reading facilitator FRS may assist a human user, or may otherwise facilitate improved reading sessions.

The radiologist referred to herein as "the user" USR in general may be compiling a radiological report which is then stored alongside or in association with the reviewed/read imagery. Report and/or imagery can be retrieved by a medical practitioner to plan a course of action, a course of treatment or further tests, etc., for the patient PAT concerned.

Broadly, the imagery $m$ is a type of spatial data that comprises measurements as pertain to different locations in 3D space, such as within the patient. The imager IA may include a signal source SS and a detector device DD. The patient may or may not rest on a patient support PS during imaging. During imaging, the signal source SS is energized to produce an interrogating signal which interacts with the patient tissue inside the patient. The so interacting radiation is modified by this interaction and is then detected by the detector device DD as a response signal, such as set of spatially resolved intensities. For example, in X-ray imaging, an X-ray beam is issued forth from the single source (in this case, an X-ray tube) which is then detected by an X-ray sensitive detector after tissue interaction. Such detectors may be of the indirect or direct conversion type. They include detector pixels based on semi-conductor materials which cause electric signals such as voltages or amperages that are proportional to the energy of the detected radiation. The radiation may be detected as intensities by the detector and may be provided by conversion circuitry in digital form, such as a set of numbers arranged in a data structure capable of recording the spatial character of the data $m$ as image elements, such as pixels or voxels in 2D or 3D matrix, for example. Higher dim ("dimensional") data, such as over-time volumetric data m, may call for higher dim data structures such as 4D tensor, etc.

Notwithstanding the above-described measurement acquisition chain, native digital acquisition, whilst preferred herein and mostly used these days, is not a necessity herein. Instead, analogue acquisition means such as X-ray sensitive film suitably arranged in a sealed container ("cassette") may, for example, be used instead, and the information so acquired therein is then post-digitized by suitable conversion equipment to obtain digital imagery $m$.

The medical modalities IA envisaged herein include tomographic imaging as well as purely projection-based imaging (radiography), such as may be used for chest X-rays or mammography. Among the tomographic modalities there are CT scanners, C-arm imagers, MRI, and nuclear imaging. Others, such as OCT and ultrasound imaging US is not excluded herein, and so are a host of other modalities such as phase contrasting imaging, dark field imaging, and others still. It will be understood that the source SS and detection device DD may having different technical realizations that in CT or X-ray: for example, in MRI, source and detector are arranged as RF coils to capture RF signals which are processed into imagery $m$. And in PET/SPECT, the detector device may be sensitive to gamma radiation as emitted by the source, in this case a (lightly) radioactive substance within patient, previously administered, etc.

As to tomographic imaging, this is based on processing projection data into volumetric data, a collection of spatially related cross-sectional slices that represent cross sections of the imaged region within the patient, referred to sometimes as the region of interest ("ROI"). It is in particular, but not only. Such volumetric imagery may be daunting and difficult to interpret as these can be reformatted into different spatial views, thus yielding a bewildering array of different visualizations. Spatial orientation in such imagery may be daunting not only for the seasoned practitioner when under stress, but mostly for novice untrained users, such as medics in residence, those in their junior years, etc.

What is proposed herein is broadly a technical solution, that is the reading facilitator system FRS, that facilitates knowledge transfer from expert(s) to the radiological novice or to less experienced user. The ability to read a medical image requires a vast amount of prior medical knowledge. The user needs to be schooled in medical technology, anatomy and pathology, etc, in order to be able to correctly ascertain or interpret ("read") the imagery. Image artifacts sometimes cloud the picture literally and it is important that the user is experienced enough to tell which structures are artifacts or bogus, and which structures are real, worthwhile further investigation. As mentioned earlier, errors of the first and second kind can have adverse consequences. As will be explored in more detail, the reading facilitator system FRS (referrable to herein simply as the "facilitator") may be based on machine learning ("ML"). Thus, whilst it has been reported in some places that machine learning may come at the expense of human jobs, the proposed system points to a different approach, one where the powers of machine learning are used in conjunction with human knowledge, for increased benefit and utility in particular in the medical art. It is the transfer to novice users of many years worth of medical knowledge, acquired by the experts in the fields, that is facilitated by the proposed system FRS. This can result in a safer, more efficient use of medical imaging. It is not only that reading errors may lead to consequences for patients, but there are other costs: for example, unnecessary image re-takes may be caused by the inexperienced reader, which may in turn put unnecessary stress and fatigue not only on the reviewer, but also on the imager IA: expensive imaging components may thus wear out prematurely, well before their time. For example, in X-ray based imaging, the anode discs in the X-ray tube are subjected to very high temperature gradients, every single time an image is taken. Excessive, and perhaps unnecessary, use of the imager IA may lead to premature failure of the anode disc, or of other components. Expensive callouts may be needed with associated inconvenience, not only in terms of cost, but also in prolonged down-time, adding yet further stress on already buckling national health care systems of some countries.

The computing system CS and, in particular the facilitator FRS, may be arranged on a single computing device, or may be distributed over plural computational entities/nodes (servers, etc.), suitably connected in a data communication network, such as in cloud or edge computing. The computing system CS and/ or the facilitator FRS may be arranged as a "for service", which can be requested by a client device for example. All or parts of the computing by facilitator FRS may be run on the end device, whilst others may be outsourced to more powerful servers or clusters of computational entities/nodes. For example, the facilitator FRS may be run at least in parts by a user end device, such as a smart phone, laptop, desktop computer, tablet or any other, whilst other part(s) may be run by a remote server or other node. The display device DID may be integrated with the computing device, such as is the case in laptops, smart phones and other such general hand-held, portable devices. In other more traditional set-ups, the workstation is arranged as a desktop computer with a processing unit coupled to a display device, as needed.

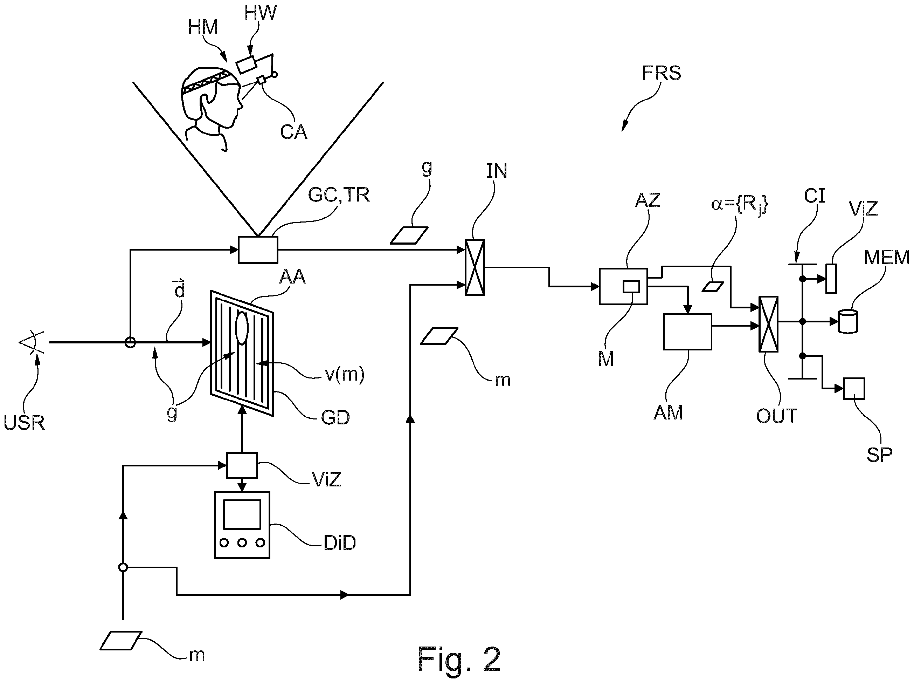

Reference is now made to Fig. 2 which shows components of the facilitator system FRS in more detail. Very broadly, the facilitator system FRS uses a machine learning model M. The model M is configured (trained) to mimic the way a radiological expert would have "looked at" (visually analyzed) a given image *m* currently displayed. The FRS may be operational whilst a current, actual, user USR attempts to read the said displayed image m. Alternatively, facilitator system FRS may be so operational during or after current user's USR reading session. The said current user/reviewer USR may possibly be an inexperienced user, such as novice radiologist USR. It is assumed herein that the current image *m* (also referred to herein as the "current image/imagery") is retrieved from any suitable source and is then visualized by visualizer on the display device DID. Thus, the display device is suitably controlled to generate a graphics display GD that includes the visualization v(m) of the medical imagery m, such of a chest X-ray or other, a tomographic slice, a rendering of at least parts of a tomographic volume, etc. The visualizer VIZ may render the visualization m(V) suitably for visual examination, e.g. in a reading room of a medical facility or elsewhere, such as in white, black and grey layout to so differentiate structures at different attenuation coefficients or in-tissue path length traversed by the radiation, etc., depending on the nature/-purpose of the medical reading task at hand. Such visualization may give rise to in-image structures possibly spatially distributed over the displayed graphics display GD as it visually may appear to current user USR on the display device's DD screen. Such visualization may render bones or other high attenuation matter as white structure, whilst low attenuation matter, such as soft tissue, air, may appear black, or as grey values. The current user USR is viewing the graphic displays GD. The eyes of the user scan the information in the visualized imagery VM, in particular for such structures, possibly spread out over the screen, thus provoking eye movement by user USR. For example, the user may gaze at an attention area on the image plane (the screen) as defined by the visualization.

A gaze capture device GC, such as an eye tracker TR, may be worn or used by the user USR whilst so viewing the imagery *m*. Such gaze capture devices have been reported previously, such as by Nachiappan Valliappan et al in "Accelerating eye movement research via accurate and affordable smart phone eye tracking". "Nature Communications", vol 11, Article number: 4553 (2020). Preferably the eye tracking afforded by such device should be sufficiently accurate, that is, the measured area of high attention should not be too smeared out over the image. Possible solution may include any one of: eye tracking function integrated in (normal / VR) glasses, integrated in screens, or even leveraging webcams, amongst others. Such or similar gaze capture device GC may include, in some constructions, a head mount HM such as a system of straps that attach to the user's head, in particular the forehead, carrying a hardware implement HW that may include suitable sensors such as an optical sensor, e.g. a camera CA. "Eye imagery" of the user USR's eyes are captured by the camera CA whilst user USR is reviewing the displayed imagery *m*. An image processer module, either integrated in the head set or remotely located to receive such eyer imagery through a suitable communication channel, image-processes the imagery to ascertain gaze information g in relation to the user's viewing action, in particular in relation to user USR's attention area. Such gaze information g may include the gaze direction J., vergence or any other ophthalmological quantity suitable to describe and define the attention area AA. Thus, the gaze information may include, or it may be inferred therefrom, a set of in-image co-ordinate(s) that define the attention area as point or a region having an in-image area. For example, an analytical geometry machine may compute intersection point of screen's plan., assuming the coordinates of the plane are known. The intersection point may then be used as, or may be expanded into the attention area AA, as needed. The term *"attention area"* will be used herein, but this should be understood herein to also include, as a "degenerated area", as a single point. The attention area may thus be any one or more of defined, stored and processed herein as a set of coordinate(s) of all pixel(s) that fall under the area AA, or such area may be defined analytically as a bound curve for example, circumscribing the attention area, such as a circle, ellipse, or box, square, etc., or in any other way.

Thus, for present purposes, gaze information g and/or the associated current user USR related attention area AA may be considered a set of pixel(s) or voxel value(s) of the currently displayed image *m.*

The gaze information g/attention area AA captured or derived by the gaze capturer GC, e.g. eye tracker TR, is dynamic data. That is, it may be provided as a time series data *g = g(t)* that varies over time with the user USR's changing gaze whilst he or she is scanning the different portions of the displayed image *m* in their reading session. Thus, gaze information *g = g(t)* is temporal data, with different associated areas AA that may change over time, caused by the user's eye movement and/or head movement. The gaze capturing device may not necessarily use camera or eye imagery as described for illustration above, but may use instead, or in addition accelerometers to ascertain the head posture of the patient and deduce therefrom the gaze information/attention area, or may use other sensors, instead or in addition, to any of the aforementioned that is suitably to be resolved into such gaze information/attention area.

Upon display of the imagery, in particular during such displaying, the gaze information g is generated and received at an input port IN of the facilitator FRS. In addition thereto, the currently displayed image *m* is also received at the same port IN, or at a different input port, not necessarily at the same time.

Broadly, an analyzer module AZ of the facilitator RS analyses the current medical image, optionally including contextual data in relation to the image, to produce an attention map $\alpha$ or similar data. This attention map $\alpha$ estimates the manner in which a seasoned practitioner, an expert in radiology, would have reviewed the given image *m.* The medical image *m* at hand may optionally be processed by analyzer module AZ alongside the said contextual data, such as information on patient, or data on the protocol or purpose for the imaging, for example that the image is intended, as a chest X-ray exam. The analyzer is capable to more robustly predict such attention data $\alpha$. In a preferred embodiment, the analyzer module AZ further analyses the gaze information g over the attention map $\alpha$ to produce monitoring or alert data in relation to the user's USR ongoing image reading session. This may be referred to herein as a real-time, "online", embodiment and this may be preferred to herein. But as an alternative, the analyzer AZ may process gaze information later over the attention map, after the user USR's viewing session, in an "offline" embodiment. The same gaze information may be used by analyzer AZ in relation to other image(s) reviewed by user USR, that are sufficiently similar to the current image. Thus, the attention map can be computed based on the image *m* alone, whether or not it is display, possibly and optionally using the contextual data which may read off metadata or may be pulled from records or are fed in by user through a user interface. Once the gaze g information is available, for which the displaying of the image *m* for user USR is needed, whenever that may be, the analysis into the monitoring or alert data may be conducted, preferably online or later as needed. The monitoring or alert data may be used herein for quality checks, such as to check or estimate whether USR has properly read the image, on which more later below.

The attention map $\alpha$, as produced by analyzer AZ's ML model M is based on training data collected in previous image reading sessions done by one or plural radiological experts, that is, human or ML based expert system, whose medical image reading knowledge is known or can be safely assumed to be greater than such reading knowledge of the current user USR. Whether or not someone qualifies as an expert can be readily and objectively established, based on exam results, PQE, or total years practicing, or other benchmarking. Preferably only data from such vetted experts is allowed to enter into the pool of training data on which model M is allowed to be trained. Indeed, when reference is made herein to the said *"expert (reviewer)", "seasoned practitioner",* or similar, this may indeed refer to single practitioner, but more often it refers to data collected in respect of a pool of such experts that have reviewed a number of similar images, not necessarily the current image. Thus, such reference should be referred to plural such experts in the image reading arts. Thus, the training data collected may represent the combined knowledge of such pool of experts. Notwithstanding the above, the definition of the term "expert (reviewer)", may be extended to any "prior reviewer/user" USR', as opposed to the current user USR, such that said users have different knowledge. For example, in some instances the current user may be the expert with greater knowledge than the prior user(s) who may now be of junior or novice knowledge level. Such a constellation may be useful, for example when expert current user USR may wish to explore for themselves a "simulation" of what makes junior radiologist "tick". Also, the level of knowledge of both, current USR and prior user USR', may be comparable, such as both being experts, and the proposed setup allows one expert USR to explore nuances of prior user(s)'s expert radiological knowledge. The proposed setup can be used in either of the above mentioned modes for knowledge exploration, with the alerts or visualizations being for information gathering purposes rather than for QCs, as the case may be.

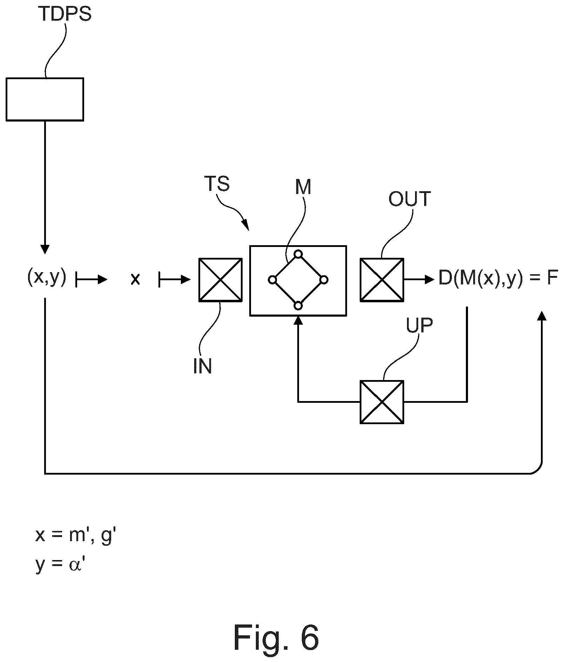

The ML training and training data collection will be explained later more fully below at Figs. 6-10. But for now, turning now first to the attention data/map $\alpha$ as such, produceable in deployment post-ML-training/ML-testing, this data $\alpha$ may be understood as spatial data, just like the image *m.* Thus, the spatial data dimension of the attention map is similar, in particular it may be equal to that of the image *m.* Data $\alpha$ may comprises entries, each associable with a different given pixel, and each such entry may represent a relative importance of a given pixel for an expert reviewer. Thus, higher values are awarded to a pixel position that was given particular attention by such expert(s). Some such importance values may be higher than the others which may give rise, after some thresholding, to certain in-image portions {Rj}. Such image portions Rj are pixel sub-sets of the image. The Ry's may be referred to herein as critical subregions or critical portions in the imagery that would have been reviewed with high likelihood by an experienced radiologist, or other expert domain. The

entries in $\alpha$ may be suitably numerically coded, such as scores, probabilities, etc., and may be obtained from class activation maps, saliency maps, etc. for a suitably trained classifier model, or similar, or may be natively obtained by a model trained specifically for $\alpha$-prediction.

The attention map $\alpha$ can be output through output port OUT. It may be communicated by the communication interface CI or other internal or external network component, a data bus, etc. for processing such as visualization by visualizer VIZ, or a different visualizer, for storage in memory MEM', or by other data consumers, such as a statistical processor SP or any other processing entity, for whatever purpose. For example, the attention map may be visually rendered in color or grey values. It may be overlaid, in a graphics overlay, on the currently displayed imagery, or may be displayed in any other manner, alongside the current imagery or on its own. It may be displayed to the possibly novice user USR, to inform them where an expert would have looked at in the given image $m$ task at hand. Occasionally, the image task may be deduced directly from the image and there is no need to supply such data in image task as separate data item.

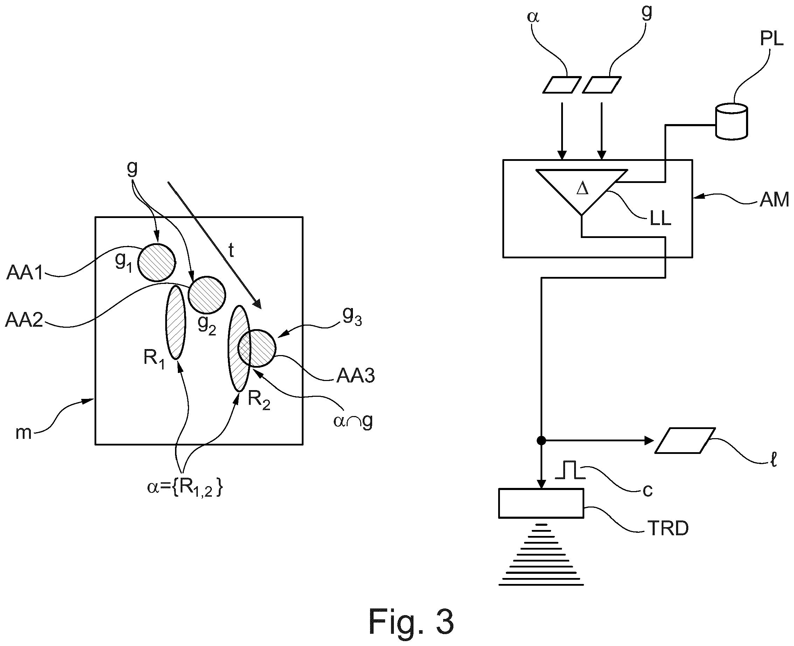

However, as said earlier, in addition or instead of such use of the attention map for displaying etc., the attention map may be processed into the said monitoring or alert data. The said processing may be done by an alert (or monitoring) module AM of facilitator FSR, as will be explained now in more detail with reference to Fig. 3. Thus, the alert (or monitoring) module AM, simply referred to herein for brevity as "the alert module AM", may be used in conjunction with the mentioned displaying of the attention map, or may be used instead, without displaying or otherwise bringing to the user's attention of the attention map $\alpha$. The alert module AM may be used in a quality check setting to acquire quality check information in relation to the current user.

Broadly, the alert module AM is operable to ascertain whether the user is reviewing the currently displayed imagery M in a manner consistent with how experienced radiologist(s) may have reviewed the image. If there is a finding that there is such a consistency, the alert module may remain dormant or issue in whichever form an "ok" signal. However, if it is found by alert module AM that there is no such consistency, then an alert signal may be issued, either in manner perceptible to the current user USR, or such signal bypasses the user, and is passed on for example to other data consumer(s) for processing at other locations. For example, the alert may be passed to a quality check department for notice. Either on site or remotely, the alert signal c so produced may be used to drive a transducer TRD to notify user USR or data consumer. For example, the alert signal my include sounding out through an acoustic transduce, or/and through a transducer of another modality, e.g., as a flash lamp, or any other. In other, more muted settings, the alert signal is written into a log-file or otherwise recorded, for later analysis or processing such by the said data consumer. The log-file may be stored for later review by suitable data consumer or may be stored in a buffer for real-time processing. The said processing may include controlling a device based on the data, or may include statistical processing for quality check ("QC") or educational processing, for monitoring, etc. The log-file or the alert data may be over-time data and the monitoring of same may be an over-time process, to allow for over-time QC of the user USR.

The operation of the alert module AM is illustrated to the left of Fig. 3. Broadly, the alert module monitors the user-caused over-time gaze information $g(t)$ against the attention map. If such monitoring reveals that a policy (e.g. one or more conditions) is violated, the alert or "ok" signal is issued. To the left of Fig. 3 there is illustrated a visualization of such monitoring operation. Specifically, there is illustrated i) the medical image $m$ as displayed, and the time series of gaze information $g=g(t),$ such as the attention areas AA1-3 over time, as may be generated by the user USR during visual reviewing of the image $m,$ and as captured by the gaze capturing device GC. The gaze information g may thus correspond to the attention areas AA1-3, which are certain pixel sub-sets of image m. The gaze information, such as such attention areas AA1-3 are compared by a logic LL of alert module AM against the attention map $\alpha= \alpha(m).$

The attention map $\alpha$ may be conceptualized, stored, or processed such as visualized, as is shown in the left of Fig. 3 as critical portions AA1-3, that is, as pixel sub-sets of the whole image $m$. Merely three such image portions are shown, but there may be more or there may be less, such as two, or a single such portion RJ, depending on the ML driven analysis of model M. The sketch to the left of Fig. 3 can be understood merely as a conceptional illustration, but such visualization may well be contemplated as a graphics display GD on display device DID, or on any other display device. However, such visualization may not be needed. It is herein to compare the in-image co-ordinates of the attention area pixels AA1-3 corresponding to the gaze information g, against the critical portions R1, R2 as per the expert attention map $\alpha$. The image sets and the gaze information and the character portions may be compared by a comparator such as logic LL. Logic LL may be operable to quantify the comparison in terms of set-theoretic operations, such as union $\cup$ and/or intersection $\cap$, such as intersection vs union, etc. for example, a ratio may be taken of the two. Dice function type formulation may be used to quantify the said comparison.

The comparison may be based on the quality check policy PL. The policy PL may be held in storage, any may be accessed and applied by the logic LL. For example, a spatial-temporal analysis may be done by logic LL of attention areas AA1-3 vs critical portions R1, R2, or the said analysis may be purely spatial or purely temporal, as the case may be. For example, the critical portions R1,2 may overlap (set theoretic intersection) at one point in time t with one or more of the attention areas AA12- corresponding to the overtime gaze information gt, g1-3. If there is no such overlap at all, at any time, it can be concluded by the logic LL that the review by reviewer USR is not in concordance with how experts would read the image, and an alert signal may be issued. However, if there is one or more such overlap at least at time(s), it may still not be sufficient, and an alert may still be issued. For example, such alert may still be warranted if the overlap area $\alpha \cap g$ may be too small given an area threshold, and/or the dwelling time of gaze information in such overlaps is too short. Either case may be indicative of a too cavalier, if not "flicking", review. Thus, the policy may prescribe certain temporal or spatial thresholds in terms of critical portion dwelling time of gaze area AA, and/or minimal overlap area between the two, in pixel numbers, length, size or other suitable measure. Having said that, a rather liberal policy may also be contemplated in which the image review by user USR is deemed of sufficient quality if there is at least some overlaps at some time.

In some embodiments, the policy PL may be based on certain baseline in-image structures obtained from expert reviews that are aggregated for consolidated consideration by the alert module. Such may also be used for calibrating and/or validating the alert module AM. For example, in lung nodule detection in chest X-rays as an example, multiple experts (eg, radiologists) USR' of various experience levels could annotate the same images. Then a baseline annotation can be aggregated per image by e.g. majority vote. The individual annotations of user can then be compared to the baseline annotations. Thus, the alert module can be calibrated / validated to issue an alert whenever user misses to detect a structure as compared to the baseline annotation, whilst on such alert is needed whenever all or most or a threshold number of baseline structures are indeed considered by the user USR.

As will be appreciated, as the critical portions Rj do not cover the whole image, there is no need in the present approach to process/check the whole image, but only parts thereof (namely the critical attention areas). This decreases computational load, and allows for better responsiveness of the system FRS - a welcome boon, given the challenging radiological workloads as mentioned above.

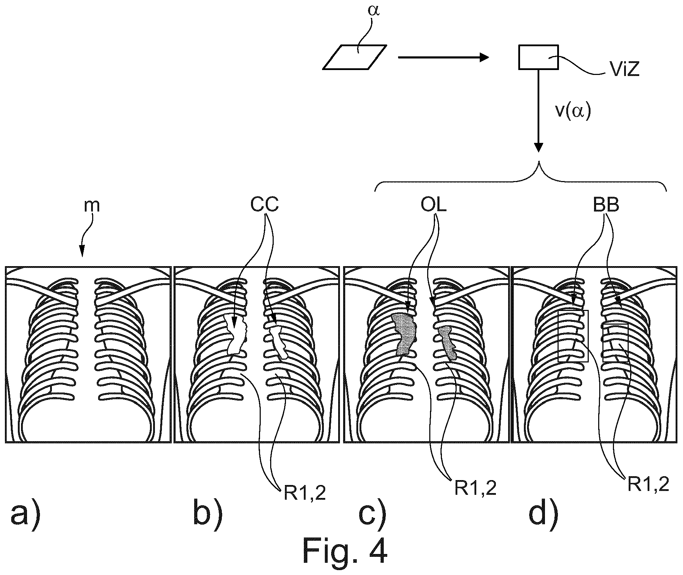

Reference is now made to Fig. 4 which shows an optional visualization $v(\alpha)$ of the attention map as produceable by visualizer VIZ, e.g. on instruction from analyzer module AZ. Fig. 4A shows schematically a visualization of the medical image $m$ as such, whilst options B-D show the same medical image $m$ in conjunction with visualization auxiliaries.

A multitude of different such visualization auxiliary are envisaged, such as overlays OL suitably color or grey value coding the critical region R1, R2 as shown in Fig. 4C, whilst Fig. 4D resorts to bounding boxes BB, or other curves, that at least partially if not fully enclose the criticality portions R1, 2. In addition, or instead, the background surrounding the critical portions R1,2 may be faded out, causing the portions R1,2 to emerge as high contrast areas CC. For example, any of such visualizations or others may be trigged by a dedicated button or hotkey in an image viewer functionality of system CS, or other. Any other manner of invoking such visualization can be configured as needed.

However, as said, such visualization may be done in conjunction or may be skipped altogether and it is only the alert module that analyses the attention map as provided by the analyzer and as explained above in Fig. 3 to issue suitable alert information, thereby ensuring certain quality standards. Thus, the facilitator in Fig. 4, and particularly in Fig. 3, can be used for educational purposes as a technical solution for efficient training and thus inter-generational knowledge transfer.

It will be appreciated that the above illustrative examples are mainly drawn from the medical field, but the principles in this disclosure are not so confined. They may be put to good use in other fields where imparting of knowledge in the interpretation of spatial data is key, such as for example in seismology or exploration, in the interpreting of radar imagery, such as in marine or aerial applications, or of sonar data as needed in manifold fields of endeavor, including engineering and others still.

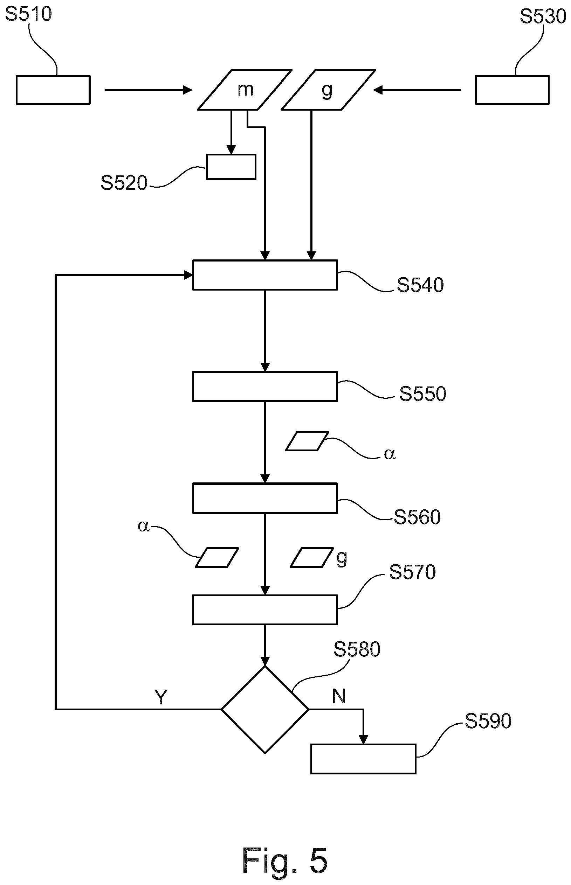

Reference is now made to Fig. 5 which shows steps of a computer aided/implemented method for facilitating reading of spatial data, e.g. of imagery or other measurements, in particular of medical imagery. The steps of the said method may be understood as implementing the above-mentioned system FRS, but below steps may also be considered instead as a teaching in their own right.

At step S510 spatial data such as medical data, in particular medical imagery tomographic or projection, or any other, is received, such as accessed in memory, e.g. a database, where such data may be held, or is forwarded from an imager that obtained the image. Alternatively, or in addition, live image feed as supplied by an imaging apparatus is received.

The medical image is then displayed at step S520 or in a graphics on a screen of the display device for viewing by a user, possibly a novice or junior user, but not necessarily.

At step S530 gaze information of the user is captured by a gaze capture device, whilst this viewer is viewing, in reading session or other, the so displayed imagery. The gaze capture device may be a wearable or may be fixedly mounted at the display device or anywhere else to acquire gaze data (e.g. eye imagery) of user in a non-ionizing manner. A time series of such gaze data may be so acquired as the user's gaze changes dynamically when scanning by eye and/head motion the displayed image for suspicious structures.

At step S540 the two data items are received, the image $m$ and the gaze data g, not necessarily at the same time, and in whichever order.

At step S550 the medical image is analyzed to predict an attention map for the image. The attention map is representative of and learned from viewing patterns from one or more experienced prior users (experts), as will be explained in more detail below at Fig. 6-10. Thus, this step S550 may be solely based on the medical image, the gaze information at this point may or may not be used.

Optionally, in addition to the medical image, other contextual data such as the imaging protocol used when image was acquired, or any data indicative of the intended medical purpose of the review may be used instead and so may be certain bio-characteristic(s) of the patient, their health record, etc., as may be pulled from medical databases, etc. Thus, the analyzer step for predicting the attention map may be purely medical image *m* based, possibly enriched with additional contextual data as an option.

A trained machine learning model may be used in the prediction of step S550.

At step S560 the attention map is made available, such as for optional visualization, in whichever form, or for other processing.

As to the latter, at step S570 is where the captured gaze information g comes into play. At that step, the gaze information as a subset of the image is compared against the attention map, in particular against critical portions (also subset(s)) of the image as per the attention map. Any one of the above (at Fig. 3) described policies may be used for this comparison. For example, spatial and/or temporal considerations may be made, such as sojourn/dwelling time of gaze, and overlap between attention areas associated with the gaze information and regions as defined by the attention map $\alpha$, such as the critical portions. To be sure, for the present purposes, whether or not the user has scanned the whole of the image as per gaze information, is irrelevant or of lesser relevance. What matters herein is a targeted, more efficient, more responsive manner, in which the imagery is analyzed. It only matters whether the gaze information comports with critical portions(s) that form only parts of the image, and whether they have been covered by the gaze information or not in sufficient manner as per the policy. Thus, the proposed method is not one for "whole image completeness", but for quality of review. A simple completeness check whether the whole image has been scanned is not what is needed herein, but such consideration may still be used in addition over and the above described, if need be.

At step S580, based on the comparison, a decision is made whether the gaze information/attention area(s) sufficiently covers the critical portion(s) as per the attention map $\alpha$. If the decision is made that it does so over, the step simply terminates, or feeds back to step S540 and awaits a new image to be reviewed as need be, or an "all clear" signal is issued etc. However, if the policy applied at step S580 concludes that no sufficient coverage was achieved, an alert signal is issued at step S590 either to the user and/or to another party and possibly unbeknownst to the user.

The above method may also be practiced dynamically instead as one-off event. Thus, it is not only that an alert signal may be issued, but the method remains operable to continue monitoring in a loop user's review activity. Thus, the reviewer may eventually cover to satisfaction the criticality portion(s), in which case the information indicates that the user now does comply with the quality requirements but did not so comply earlier when the alert signal was issued. Similarly, once user compliance has established over time, such alert module can still be issued in relation to a new image reviewed by the same reviewer, which review may not be of quality, although it so was for an earlier reviewed image.

The above Figs. 1-5 primarily pertained to deployment of model M post-training. The below explores now in more details aspects of training such model.

In this connection, reference is now made first to Fig. 6 which shows a training system TS as may be used to train the machine learning model M for use in the prediction of attention maps $\alpha$, such as by analyzer module AZ mentioned above. A training data provider system TDPS may provide the necessary training data denoted herein as *(x,y)* in order to distinguish the training data from the deployment data mentioned earlier as both data sets, whilst similar, appertain to different instances of a patient etc. The model *M* may be trained in a training phase prior to deployment or testing phase. The above at Figs. 1-5 pertained to deployment/inference or testing, thus, post-training. In contrast, the below pertains to training, which is prior to deployment or testing. Thus, the data set (m, $\alpha$) as used above is different from the training data *(x,y)*, although the two may be statistically similar. For example, both sets can be statistically characterized as having been drawn from the same or similar distributions, and indeed such may be useful for good performance of the trained model in practice during deployment/inference. The training phase may be a one-off, or may be repeated periodically or when appropriate, such as when new or more varied, etc., training data emerges.

The training data set ("corpus") comprises training input data x, and associated target/ ground truth data *y*. Both data may be pulled from historical data that may be held in health records, or imaging databases, PACSs, etc. Ground truth *y* may include expert attention maps $\alpha'$ as collected by the training data provider systems TDPS as will be explained in more detail below, whilst the training input data x may include historical imagery and gaze information g', whichever way such data was obtained. However, it may also be possible to synthetically generate such training data such as based on generative ML models, or, as proposed herein in embodiments, may be generated in field trials setup at a given medical facility where the model *M* ought to be used post-training.

In general, a supervised training setup may be used, but the principles described herein may not be so confined. Other training setups, such as semi-supervised or self-leaning, or reinforced learning schemes may be used in addition.

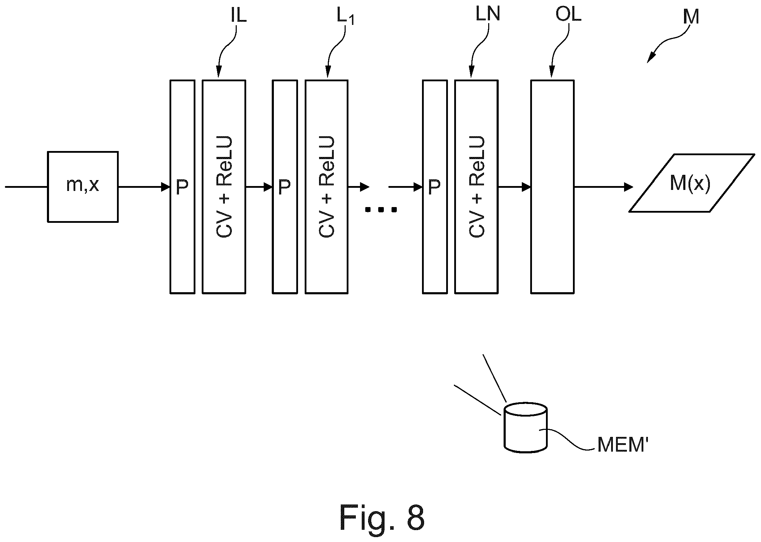

In training phase, the training system TS is operable to adapt parameters of model *M* Initially, model *M* is pre-populated with an initial set of parameters. The parameters $\theta$ of the model *M* represent a parameterization $M^{\theta}$ of model *M*. The parameters may include weights or filter parameters, such as in convolutional operators that are part of the model architecture (see Fig. 8 below for more detail). It is the object of the training system TS to optimize and hence adapt the parameters $\theta$ based on the training data pairs $\{(x_i, y_i)'\}$. In other words, the learning can be formulized mathematically as an optimization scheme where a cost function *F* is minimized, although the dual formulation of maximizing a utility function may be used instead.

Assuming for now the paradigm of a cost function *F*, this measures the aggregated residue(s), that is, the summed error incurred between data estimated by the model M$^\theta$ given the current parameter $\theta$, and the targets as per some ,or all, of the training data pairs *i* in a batch or over all training data:

$$argmin_\theta F = \sum_k D[M^\theta(x_k), y_k] \qquad (1)$$

In eq. (1), function M$\theta$() denotes the result of the model M applied to training input x. The result will differ in general from the associated target *y*. This difference, or the respective residuals for each training pair *i*, are measured by a distance measure *D*[.,.]. Thus, the cost function *F* may be pixel/voxel-based, such as the L1 or L2-norm cost function, or any other norm L*p*. Specifically, The Euclidean-type cost function in (1) (such as least squares or similar) may be used for regression task, such as when output layer of a neural network model regresses into attention map. When the model is to act as classifier, the summation in (1) is formulated instead as one of cross-entropy or Kullback-Leibler divergence or similar. Binary or multi-class classification is envisaged herein.

An optimization procedure such as backward/forward propagation or other gradient based method may then be used to adapt the parameters $\theta$ of the model M to so improve (e.g., decrease) objective function output *F()*.

The optimization procedure may proceed iteratively. After one or more iterations in a first, inner, loop in which the parameters $\theta$ of the model are updated by updater UP for the current batch of pairs, the training system TS enters a second, an outer, loop where a next training data pair or a next batch is processed accordingly. The structure of updater UP depends on the optimization procedure used. For example, the inner loop as administered by updater UP may be implemented by one or more forward and backward passes in a forward/backpropagation algorithm or other gradient based setup, based on the gradient of *F*. Each set ("batch") comprising plural training data items and the summation in (1) extends over the whole respective batch, rather than iterating one by one through the training pairs, although this latter option is not excluded herein.

GPU(s) maybe used to implement the training system TS. The fully trained machine learning module M may be stored in one or more memories MEM', and can be made available as trained model, such as for use in system FRS as described herein.

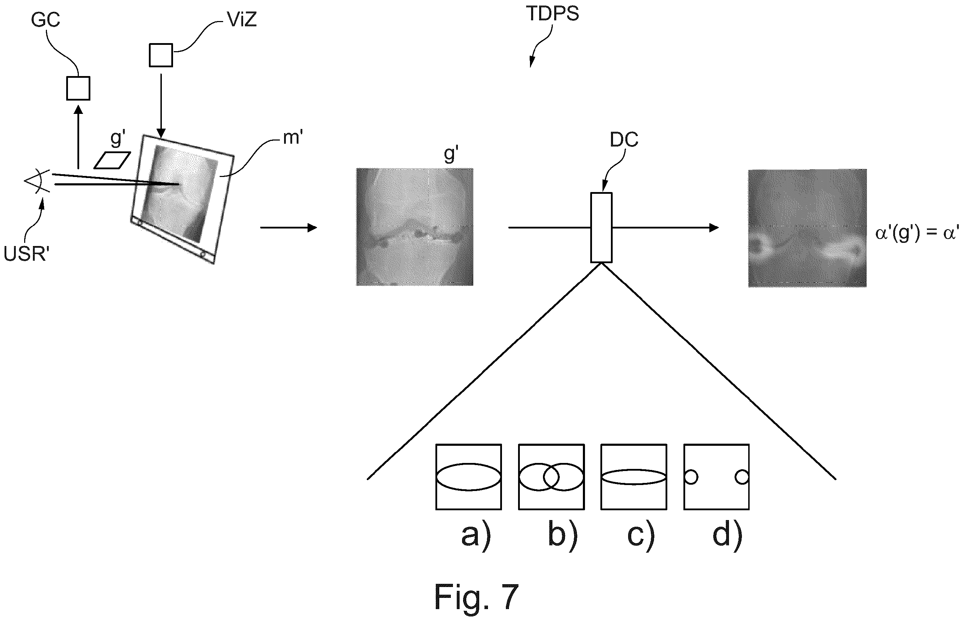

Reference is now made to Fig. 7, which illustrates in more detail operation of the training data provider system TDPS. The general set-out is similar to the one shown in Fig. 2: it may comprise a viewing station, with a display device on which historical imagery *m'* from prior exams is displayed to an expert/prior user USR' in a field trial or experimental training data collection event. Again, a gaze capture device GC is used, not necessarily of the same type, model or make as in deployment (Figs. 1-5). Thus, the same procedure was done in the training data collection trail as outlined earlier above at Figs. 1-5 in deployment, only that then the gaze information g's was collected from expert /prior user USR', instead of for current user URS in deployment. The expert gaze data *g'* is stored as expert attention map $\alpha'$ as target *y*, in association with the imagery *m'* considered as training input *x*. Thus, the training data *(x=m', y= $\alpha'$(g'))* is defined. Collection of such pair *(x,y)* is repeated for multiple experts and/or multiple historical imagery acquired for multiple purposes to so build up a corpus *{(x,y)$^i$}* of different specimens *i*, in sufficient variation and number *|{(x,y)$^i$}|*. Said number may be in the 100s or 1000s, as needed. Instead of running the above data collection as a trial, the use of the data capture device GC may be simply integrated into every day clinal image reading sessions and practice, with only minimal discomfort for readers USR': expert readers USR', who are in possession of requisite experience credentials are simply asked to use such capture device GC over a sufficient long period of time until such corpus *{(x,y)$^i$}* of said sufficient variation and number (data quality) accumulates. An accompanying statistical monitoring may be done over the accumulated corpus, and once the asked of data quality is attained, training can be initiated, e.g. as in Fig. 6 for example, or in any other appropriate way. If context data is to be used, such may be pulled from records, and may be adjoined or otherwise associated with the respective pair.

Optionally, but preferably, a data conditioner DCON is used that processes the collected gaze information *g'* from the expert into cleansed attention maps $\alpha'$ (the same notion "$\alpha$'" is used herein for the cleansed data). This is because saccade movements of the eye may make the attention map indistinct. Saccade points as may be recorded by the gaze capture device GC during viewing do not usually count as parts of the attention/fixation areas. Rather, such saccade points are inter-attention-area points, traced out over the image plane as user USR's visually scans from one attention area to the next. Thus, it is preferred herein that such saccade points are removed by cleansing as illustrated in at the foot of Fig. 7 at inlays A)-D) to so separate fixation points from saccade phases/point. For example, Fig. 7A) illustrates a collection of "raw" gaze information points as collected, without cleansing. However, because of the many saccade points, the attention map shown in 7B) is indistinct, without clear definition of critical regions. For example, the regions may overlap, to form a partly merged structure. Such a situation "tainted" by saccade points may make use of such gaze information difficult. It may make operation of the alert module more difficult, with sub-par performance. Effects of data cleansing, in particular saccade points removal, are illustrated in Figs. 7C), D). The gaze point cloud is now narrower, including fewer points (Fig. 7C)), which yields a proper separation (Fig. 7D)) of the critical regions, e.g. after thresholding.

Stochastic process modeling may be used to separate fixation points that form the attention/fixation areas from such saccade points. This can be achieved by e.g., modelling eye dynamics by a stochastic process, such as Levy flights or as other random walks. A stochastic process-based approach using Levy flights was described by Wang, Sheng, et al. in "Follow my eye: Using gaze to supervise computer-aided diagnosis", published in IEEE Transactions on Medical Imaging, vol 41.7 (2022), pp 1688-1698. Any other outliner detection scheme may be used herein instead, or in addition, to saccade cleansing. In addition, or instead of saccade cleansing, conditioning may include filtering, noise removal, etc. For example, after such cleansing by saccade point removal, the so processed gaze points may be converted into a smoothened attention map $\alpha'$, using e.g., a kernel-density-estimation technique.

The above setup allows obtaining training data for the attention-map prediction algorithm without manual annotations. If the training input image $x=m'$ is a tomographic 3D volume (e.g., a CT or MR scan), the gaze points may be recorded per slice and accordingly stored.

Use of such data conditioner DCON, such as for saccade removal and optional smoothing, may also be envisaged during deployment as an option. However, use of this merely in the training phase may be sufficient in some cases.

Reference is now made to Fig. 8 which shows an architecture of a machine learning model M as may be used herein, for example a machine learning model of the neural network type NN may be used, in particular of the convolutional network type ("CNN").

In general, the NN model M is made up of a set of computational nodes arranged in cascading layers, with nodes in one layer passing their output as input to nodes in a follow up layer

CNNs may include use of convolutional filters CV arranged in hidden (one or more) layers $Lj$ between an initial input layer $IL$ and a final output layer $OL$. Such convolutional networks, or at least partly convolutional networks, have been found to be useful in processing spatial data, such as the input imagery $x=m'$. In general, a regressor or classifier architecture can be used. In some hybrid architecture, there are convolutional and fully connected layers. For example, the last, or the last few, layers prior output layer OL may be arranged as fully connected layers following-on on earlier layers which may convolutional. In classification set-ups the last layer OL is a combination layer. The output layer OL may be a soft-max layer which combines the feature maps from an earlier layer into the desired classification, such as into map $\alpha'$.

Some or all of the hidden layers are convolutional layers, that is, include one or more convolutional filters CV which process an input feature map from an earlier layer into intermediate output, sometimes referred to as logits. An optional bias term may be applied by addition for example. An activation layer processes in a non-linear manner the logits into a next generation feature map which is then output and passed as input to the next layer, and so forth. The activation layer may be implemented as a rectified linear unit RELU as shown, or as a *soft-max*-function, a sigmoid-function, tanh-function or any other suitable non-linear function. Optionally, there may be other functional layers such as pooling layers P or drop-out layers (not shown) to foster more robust learning. The pooling layers P reduce dimension of output whilst drop-out layer sever connections between nodes from different layers. The model $M^{\theta}$, in particular its trained parameters $\theta$ may be held in a memory MEM' for later use.

The layers of the network, and indeed the input $x$ and output $y$, and the intermediary input and output between hidden layers (referred to herein as feature maps), can be represented as two or higher dimensional matrices ("tensors") for computational and memory allocation efficiency.

Instead of NN type network, other models may be used such as support vector machines (SVM), decision trees, etc.

The model M may be trained to produce the attention maps $\alpha$ end-to-end. Thus, input imagery is classified or regressed into such attention maps $\alpha$. Alternatively, certain auxiliaries are used as "bolt-ons", such as class activation maps CAM, or saliency maps, for already trained model. Such auxiliaries, "tap" into existing, already trained models and ascertain which regions in a given input image x contributed more than others to the outcome y, such as a disease classification given a training image applied to an already trained model. Gradient based techniques, or more general techniques of numerical sensitivity analysis (eg, condition numbers, etc) may be used to so ascertain such regions. The so ascertained regions may then be provided as the attention maps sought.

If use of the context data is intended, this can be processed alongside the training input $x=m'$. in different input data channels, such as in the neural network for example.

The observations and manner of processing of NN networks or others as explained herein in connection with Fig. 6 are equally applicable to training and deployment/testing, except that the model parameters $\theta$ are different.

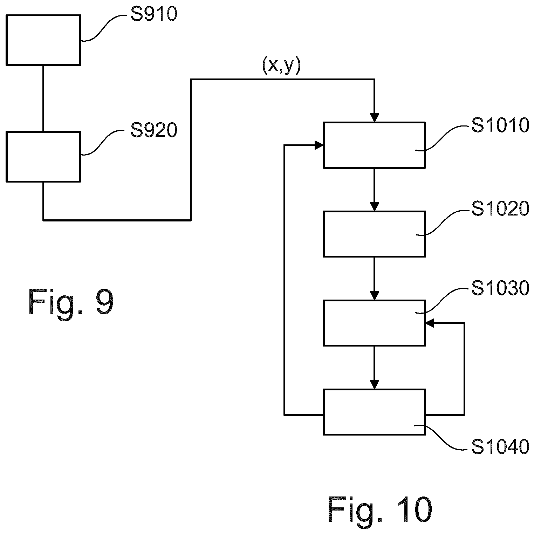

Reference is now made to Fig. 9 which shows a flow chart of a method of providing training data.

At step S910 historical or synthetically generated imagery $x=m'$ is accessed.

At step S920. based on imagery, there is produced attention maps $y=\alpha'$ by experts as ground truth versus the input imagery x. The target attention maps y may be collected from experts in their reading sessions.

Specifically, the ground truth/target data y may be produced as explained above in Fig. 7 by monitoring expert reading sessions and recording associated attention maps by suitable gaze captured device that may be mobile/wearable or fixed. Such gaze capture devices may be worn by expert user USR', and are operable during their review. Instead of being wearables, gaze captured devices may be fixedly installed, such as cameras mounted around the display device for example or in any other way suitable to capture eye imagery. Instead of, or in addition to, image sensors, accelerometer sensors may be used instead to capture gaze data y.

The attention maps *y* so collected are then collated or otherwise associated with the input imagery x reviewed, to so obtain a set of pairs (*x,y*) which may then serve as training input data, as explained above.

Reference is now made to Fig. 10 which shows a flow chart of a method of training a machine learning model based on the training data so provided or otherwise sourced.

At step S1010 the training data is received. This may data (x,y) as provided by the method of Fig. 9, or in whichever way.

Broadly, based on the training data, parameters of the model are adapted. This adaptation may be done in an iterative optimization procedure. The procedure is driven by objective function F. Once a stopping condition is fulfilled, the model is considered trained.

In more detail, and with particular reference to a supervised training setup, at step S1020, the training inputs x in the current batch are applied to a machine learning model M having current parameters $\theta$ to produce a training outputs $M(x)$.

A deviation, or residue, of the training output $M(x)$ from the respective associated target *y* is quantified at S1030 by cost function *F*. One or more parameters of the model are adapted at step S1040 in one or more iterations in an inner loop to improve the cost function. For instance, the model parameters are adapted to decrease residues as measured by the cost function. The parameters may include in particular weights of the convolutional operators, in case a convolutional NN model M is used.

The training method then returns in an outer loop to step S1010 where the next batch of training data is fed in. In step S1020, the parameters of the model are adapted so that the aggregated residues, considered over the current and preferably over some or all previous batches are decreased, in particular minimized. The cost function quantifies the aggregated residues. Forward- backward propagation or similar gradient-based techniques may be used in the inner loop. A dual formulation in terms of a maximization of a utility function is also envisaged.

Examples for gradient-based optimizations may include gradient descent, stochastic gradient. conjugate gradients, Maximum likelihood methods, EM-maximization, Gauss-Newton, and others. Approaches other than gradient-based ones are also envisaged, such as Nelder-Mead, Bayesian optimization, simulated annealing, generic algorithms, Monte-Carlo methods, and others still.

The components of the system FRS may be implemented as one or more software modules, run on one or more general-purpose processing units PU such as a workstation associated with the imager IA, or on a server computer associated with a group of imagers.

Alternatively, some or all components of the system FRS may be arranged in hardware such as a suitably programmed microcontroller or microprocessor, such an FPGA (field-programmable-gate-array) or as a hardwired IC chip, an application specific integrated circuitry (ASIC), integrated into the imaging system MDRA. In a further embodiment still, the system FRS may be implemented in both, partly in software and partly in hardware.

The different components of the system FRS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1.    A system (FRS) for facilitating radiological reading of medical imagery, comprising:

    an input interface (IN) for receiving input data including a medical image to be read by a user;
    an analyzer (AZ) capable of identifying, based on the medical image (m), one or more critical portions (R1-3); and
    an alert module (AM) capable of issuing an alert signal, based on the so identified one or more critical portions (R1-3), and on user generatable gaze information (g) in respect of the said medical image displayable on a display device (DD).

2.    System of claim 1, wherein a criticality of said one or more portions is based on reading of prior imagery by prior one or more users.

3.    System of claim 1 or 2, wherein the issuing of the alert signal is based on the alert module (AM) comparing the said one or more critical portions (R1-3) against the said gaze information.

4.    System of claim 3, wherein the said alert module (AM), in so comparing, is capable to consider one or more factors including any one or more of: i) gaze dwelling time over the one or more critical portions (R1-3), ii) number of the one or more critical portions (R1-3) over the gaze information.

5.    System of any one of preceding claims, wherein analyzer (AZ) is based on a trained machine learning model.

6.    System of any one of preceding claims, wherein at least one or more of the said identified one or more critical portions (R1-3) is displayable on the, or a, display device (DD).

7.    System of any one of preceding claims, wherein the gaze information is acquirable by a tracker system.

8.    System of claim 7, wherein tracker system includes a headset wearable by user during reading.

9. Arrangement (MDRA) comprising a system of any one of preceding claims, and further comprising any one of more of: i) display device, ii) the tracker system, iii) an imaging apparatus (IA) providing the medical image, iv) a database from (MEM) which the medical image is retrievable.

10. Training system (TS) for training, based on training data, the model of claim 5.

11. A training data provider system (TDPS) capable of providing the training data for training of model by training system of claim 10.

12. The training data provider system (TDPS) of claim 11, capable of capturing gaze information from prior one or more users, whilst said prior one or more users view prior imagery, the said gaze information forming part of the training data.

13. Method for facilitating radiological reading of medical imagery, comprising: receiving (S510) input data including a medical image to be read by a user;

identifying (S550), based on the medical image (m), one or more critical portions (R1-3); and issuing (S580) an alert signal, based on the so identified one or more critical portions (R1-3), and on user generatable gaze information (g) in respect of the said medical image displayable on a display device (DD).

14. A computer program element, which, when being executed by at least one processing system, is adapted to cause the processing system to perform the method as per claim 13.

15. At least one computer readable medium having stored thereon the program element of claim 14, or having stored thereon the machine learning model (M) as per claim 7.

SS

IA

MDRA

CS

PAT

CI

MEM

PS

XB

DD

m

ViZ

DID

DST

FRS

# Fig. 1

Fig. 2

Fig. 3

Fig. 4

S510

S530

m    g

S520

S540

S550

α

S560

α    g

S570

S580

Y    N

S590

Fig. 5

TDPS

TS   M   OUT

$(x,y) \longmapsto$   $x \longmapsto$   ⊠   ◇   ⊠   $\rightarrow D(M(x),y) = F$

IN   UP   ⊠

$x = m', g'$
$y = \alpha'$

## Fig. 6

Fig. 7

Fig. 8

S910

S920

(x,y)

Fig. 9

S1010

S1020

S1030

S1040

Fig. 10

# EP 4 675 634 A1

Europäisches
Patentamt
European
Patent Office
Office européen
des brevets

# EUROPEAN SEARCH REPORT

**Application Number**

**EP 24 18 5914**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/087724 A1 (SAWARKAR ABHIVYAKTI [US] ET AL) 14 March 2024 (2024-03-14) | 1-7,9, 10,13-15 | INV. G16H30/40 |
| Y | * paragraphs [0003], [0004], [0030], [0043]; figures 1, 6 * | 8,11,12 | G16H50/20 |
| Y | US 11 054 903 B2 (TOBII AB [SE]) 6 July 2021 (2021-07-06) * figure 6 * | 8,11,12 | |
| A | WANG SHENG ET AL: "Follow My Eye: Using Gaze to Supervise Computer-Aided Diagnosis", IEEE TRANSACTIONS ON MEDICAL IMAGING, IEEE, USA, vol. 41, no. 7, 26 January 2022 (2022-01-26), pages 1688-1698, XP011912989, ISSN: 0278-0062, DOI: 10.1109/TMI.2022.3146973 [retrieved on 2022-01-27] * the whole document * | 1-15 | |

**TECHNICAL FIELDS SEARCHED (IPC)**

G16H

The present search report has been drawn up for all claims

| Place of search | Date of completion of the search | Examiner |
|---|---|---|
| Munich | 12 December 2024 | Schmidt, Karsten |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## EP 4 675 634 A1

### ANNEX TO THE EUROPEAN SEARCH REPORT
### ON EUROPEAN PATENT APPLICATION NO.

EP 24 18 5914

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2024

| Patent document cited in search report | Publication date | Patent family member(s) | Publication date |
|---|---|---|---|
| US 2024087724 A1 | 14-03-2024 | CN 116709986 A<br>EP 4272221 A1<br>US 2024087724 A1<br>WO 2022144360 A1 | 05-09-2023<br>08-11-2023<br>14-03-2024<br>07-07-2022 |
| US 11054903 B2 | 06-07-2021 | CN 112148119 A<br>US 2021011551 A1 | 29-12-2020<br>14-01-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **R ALEXANDER et al.** Mandating Limits on Workload, Duty and Speed in Radiology. *Radiology*, vol. 304, 274-282
- **T. M. MITCHELL**. Machine Learning. McGraw-Hill, 1997, 2, 6-1.2.1

- **NACHIAPPAN VALLIAPPAN et al.** Accelerating eye movement research via accurate and affordable smart phone eye tracking. *Nature Communications*, 2020, vol. 11
- Follow my eye: Using gaze to supervise computer-aided diagnosis. **WANG, SHENG et al.** IEEE Transactions on Medical Imaging. 2022, vol. 41, 1688-1698